# EUROPEAN PATENT SPECIFICATION

(11) **EP 0 968 197 B1**
(45) Date of publication and mention of the grant of the patent: **27.04.2005**
(21) Application number: 98906255.9
(22) Date of filing: 09.02.1998
(51) Int. Cl.: C07D 265/18, C07C 271/28

(54) **EFFICIENT SYNTHESIS OF A 1,4-DIHYDRO-2H-3,1-BENZOXAZIN-2-ONE**
WIRKSAMES VERFAHREN ZUR HERSTELLUNG VON 1,4-DIHYDRO-2H-3, 1-BENZOXAZIN-2-ON
SYNTHESE EFFICACE DE 1,4-DIHYDRO-2H-3,1-BENZOXAZIN-2-ONE

(30) Priority: 12.02.1997 US 37059 P; 24.03.1997 GB 9706082
(43) Date of publication of application: 05.01.2000
(73) Proprietor: MERCK & CO., INC., Rahway, New Jersey 07065 (US)
(72) Inventor: FREY, Lisa, F., Rahway, NJ 07065 (US); TILLYER, Richard, D., Rahway, NJ 07065 (US); GRABOWSKI, Edward, J., J., Rahway, NJ 07065 (US)
(74) Representative: Horgan, James Michael Frederic
(86) International application number: PCT/US1998/002495
(87) International publication number: WO 1998/034928

(56) References cited:
- WO-A-96/37457
- M. UCHIDA ET AL.: "Synthesis and Antiulcer Activity of 4-Substituted 8-[(2-Benzimidazolyl)sulfinylmethyl]-1,2,3 ,4-tetrahydroquinolines and Related Compounds" CHEMICAL & PHARMACEUTICAL BULLETIN, vol. 38, no. 6, 1990, pages 1575-86, XP002065416
- GOUILLEUX L ET AL: "Solid Phase Synthesis of chiral 3-substituted Quinazoline-2,4 -diones" TETRAHEDRON LETTERS, vol. 39, no. 37, 23 September 1996, page 7031-7034 XP004030817
- : "", CHEMISTRY LETTERS, , 1973, vol. , no. , pages 1143 to
- : "", J. ORG. CHEM. , , 1985, vol. 50, no. , pages 715 to

## Description

### BACKGROUND OF THE INVENTION

A key step in the synthesis of the reverse transcriptase inhibitor, (-)-6-chloro-4-cyclopropylethynyl-4-trifluoromethyl-1,4-dihydro-2H-3,1-benzoxazin-2-one, also known as DMP-266, is the cyclization of the amino alcohol using phosgene.

The synthesis of DMP-266 and structurally similar reverse transcriptase inhibitors are disclosed in US Patent 5,519,021 and the corresponding PCT International Patent Application WO 95/20389, which published on August 3, 1995. Additionally, the asymmetric synthesis of an enantiomeric benzoxazinone by a highly enantioselective acetylide addition and cyclization sequence that has been described by Thompson, *et al*., Tetrahedron Letters *1995*, 36, 8937-8940, as well as the PCT publication, WO 96/37457, which published on November 28, 1996.

The instant invention discloses an efficient method for the cyclization of an amino alcohol of formula 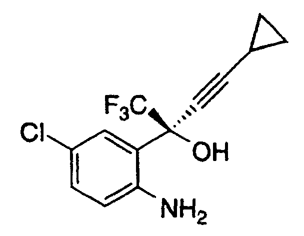 using a chloroformate and a base in a solvent to give the 1,4-dihydro-2H-3,1-benzoxazin-2-one

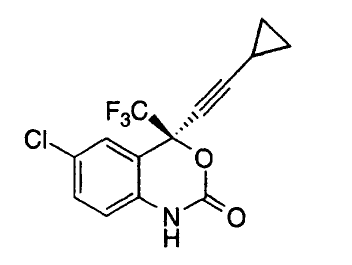

This cyclization method employs an aryl or alkyl chloroformate and avoids the use of phosgene, a toxic and highly hazardous gas, which requires special handling.

### SUMMARY OF THE INVENTION

The instant invention relates to an efficient method for the cyclization of an amino alcohol of formula 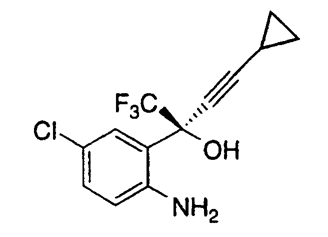 using a chloroformate and a base in a solvent to give the 1,4-dihydro-2H-3,1-benzoxazin-2-one

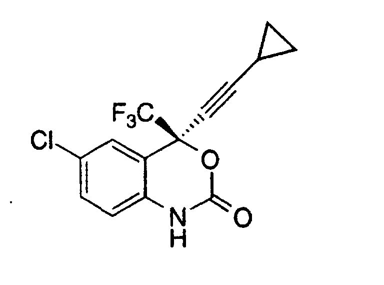

### DETAILED DESCRIPTION OF THE INVENTION

The instant invention discloses an efficient process for the preparation of a 1,4-dihydro-2H-3,1-benzoxazin-2-one of the formula 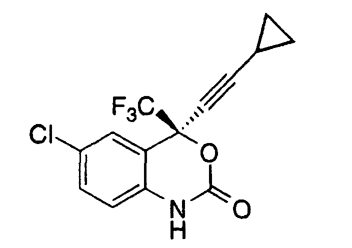 comprising the steps of:
1) adding an aryl chloroformate to a stirring mixture of an amino alcohol of formula 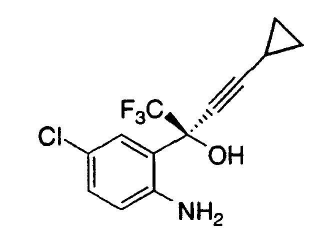 in an organic solvent with a base at a temperature of about 0°C to about 25°C under an inert atmosphere to produce a carbamate intermediate of formula 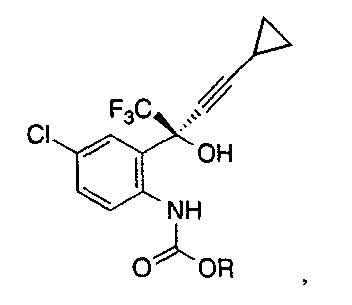 wherein R represents the aryl side chain of the chloroformate;
2) stirring the reaction mixture at about 20°C to about 25°C for about 1 to about 6 hours to complete the formation of the carbamate intermediate;
3) quenching the reaction with water or an aqueous base to produce a biphasic solution containing the 1,4-dihydro-2H-3,1-benzoxazin-2-one in the organic solvent phase;
4) stirring the biphasic mixture at about 20°C to about 50°C for about 1 to about 6 hours to complete the cyclization to the 1,4-dihydro-2H-3,1-benzoxazin-2-one; and
5) isolating the 1,4-dihydro-2H-3,1-benzoxazin-2-one from the organic phase.

The process for the preparation of a 1,4-dihydro-2H-3,1-benzoxazin-2-one as recited above, wherein the aryl group of the aryl chloroformate is defined as phenyl or naphthyl, which is optionally substituted with one, two or three substituents selected from the group consisting of: halo (F, Cl, Br, I), CF₃, CO₂C₁-C₆-alkyl, and NO_{2.} An embodiment of the aryl chloroformates useful in this process are defined as phenyl chloroformate, in which the phenyl is optionally substituted with one, two or three substituents selected from the group consisting of: halo (F, Cl, Br, I), CF₃, and NO₂. A preferred aryl chloroformate useful in the process is 4-nitrophenyl chloroformate.

The process for the preparation of a 1,4-dihydro-2H-3,1-benzoxazin-2-one as recited above, wherein the base is defined as a solid or solution of KOH, NaOH, LiOH, K₂CO₃, Na₂CO₃, Li₂CO₃, KHCO₃, NaHCO₃, LiHCO₃, or a combination of said bases. The process for the preparation of a 1,4-dihydro-2H-3,1-benzoxazin-2-one as recited above, wherein the preferred base is defined as a solid or solution of KOH, NaOH, K₂CO₃, Na₂CO₃, KHCO₃, NaHCO₃, or a combination of said bases.

The process for the preparation of a 1,4-dihydro-2H-3,1-benzoxazin-2-one as recited above, wherein the organic solvent is selected form the group consisting of: methyl t-butyl ether, toluene, tetrahydrofuran, acetonitrile dimethylacetamide, N-methylpyrrolidinone, or a combination of said solvents.

A preferred embodiment of the instant invention is the process for the preparation of a 1,4-dihydro-2H-3,1-benzoxazin-2-one of the formula 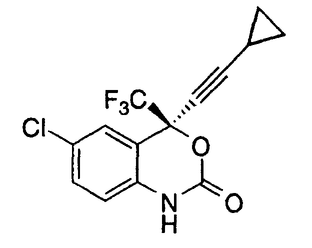 comprising the steps of:
1) adding 4-nitrophenyl chloroformate in batches to a stirring mixture of an amino alcohol of formula 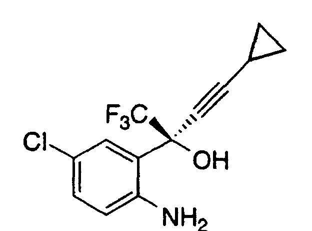 in methyl tert-butyl ether with an aqueous solution of KHCO₃ at a temperature of about 25°C under a nitrogen atmosphere maintaining a pH of between about 8.5 and 4 to produce a carbamate intermediate of formula 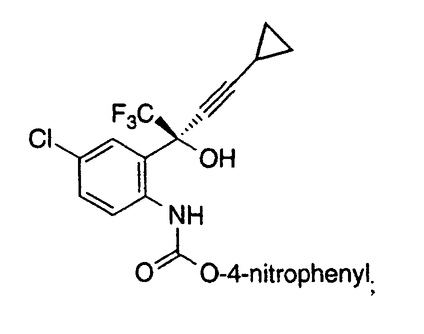
2) stirring the reaction mixture at about 20°C to about 25°C for about 2 hours to complete the formation of the carbamate intermediate;
3) quenching the reaction with an aqueous KOH to a pH of about 11 and adding water to produce a biphasic mixture containing the 1,4-dihydro-2H-3,1-benzoxazin-2-one in the organic solvent phase;
4) isolating the 1,4-dihydro-2H-3,1-benzoxazin-2-one from the organic phase.

A process for the preparation of a 1,4-dihydro-2H-3,1-benzoxazin-2-one of the formula 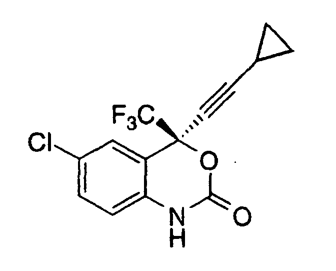 comprising the steps of:
1) adding an aryl chloroformate to a stirring mixture of an amino alcohol of formula 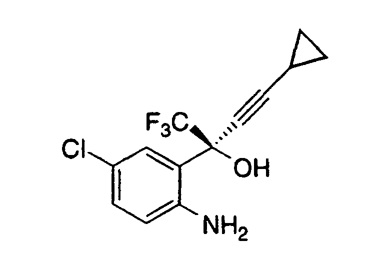 in an organic solvent at a temperature of about 0°C to about 25°C under an inert atmosphere to produce a carbamate intermediate of formula 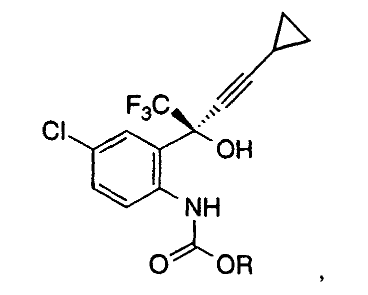 wherein R represents the aryl side chain of the chloroformate;
2) stirring the reaction mixture at about 20°C to about 25°C for about 1 to about 6 hours to complete the formation of the carbamate intermediate;
3) quenching the reaction with an aqueous base to produce a biphasic solution containing the 1,4-dihydro-2H-3,1-benzoxazin-2-one in the organic solvent phase;
4) stirring the biphasic mixture at about 20°C to about 50°C for about 1 to about 6 hours to complete the cyclization to the 1,4-dihydro-2H-3,1-benzoxazin-2-one; and
5) isolating the 1,4-dihydro-2H-3,1-benzoxazin-2-one from the organic phase.

The process for the preparation of a 1,4-dihydro-2H-3,1-benzoxazin-2-one as recited above, wherein the aryl group of the aryl chloroformate is defined as phenyl or naphthyl, which is optionally substituted with one, two or three substituents selected from the group consisting of: halo (F, Cl, Br, I), CF3, CO₂C₁-C₆-alkyl, and NO_{2.}

The process for the preparation of a 1,4-dihydro-2H-3,1-benzoxazin-2-one as recited above, wherein the base is defined as a solid or solution of KOH, NaOH, LiOH, K₂CO₃, Na₂CO₃, Li₂CO₃, KHCO₃, NaHCO₃, LiHCO₃, or a combination of said bases.

The process for the preparation of a 1,4-dihydro-2H-3,1-benzoxazin-2-one as recited above, wherein the organic solvent is selected form the group consisting of: methyl t-butyl ether, toluene, or a combination of said solvents.

The process for the preparation of a 1,4-dihydro-2H-3,1-benzoxazin-2-one as recited above, wherein the aryl group of the aryl chloroformate is defined as phenyl chloroformate, in which the phenyl is optionally substituted with one, two or three substituents selected from the group consisting of: halo (F, Cl, Br, I), CF₃, and NO_{2.}

The process for the preparation of a 1,4-dihydro-2H-3,1-benzoxazin-2-one as recited in above, wherein the base is defined as a solid or solution of KOH, NaOH, K₂CO₃, Na₂CO₃, KHCO₃, NaHCO₃, or a combination of said bases.

The process for the preparation of a 1,4-dihydro-2H-3,1-benzoxazin-2-one as recited above, wherein the aryl group of the aryl chloroformate is defined as 4-nitrophenyl chloroformate.

Another aspect of the instant invention is the process for the preparation of a 1,4-dihydro-2H-3,1-benzoxazin-2-one of the formula 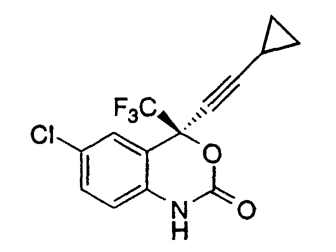 comprising the steps of:
1) adding an alkyl chloroformate to a stirring mixture of an amino alcohol of formula 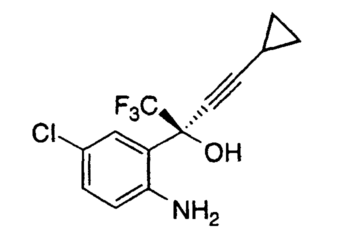 in a first organic solvent with a first base at a temperature of about 0°C to about 25°C under an inert atmosphere to produce a carbamate intermediate of formula 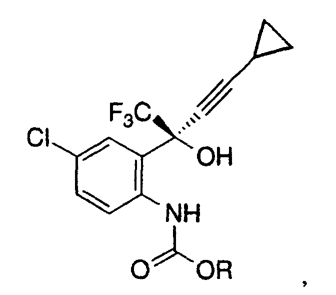 wherein R represents the alkyl side chain of the chloroformate;
2) stirring the reaction mixture at about 20°C to about 25°C for about 1 to about 30 hours to complete the formation of the carbamate intermediate;
3) isolating the organic phase containing the alkyl carbamate;
4) distilling about 90% to about 95% of the first organic solvent in vacuum and adding a counter solvent to isolate the solid alkyl carbamate;
5) adding a second organic solvent to the solid alkyl carbamate to form an alkyl carbamate solution;
6) reacting the alkyl carbamate solution with a second base at a temperature range of about 20°C to about 25°C for about 2 hours to about 30 hours to produce the 1,4-dihydro-2H-3,1-benzoxazin-2-one;
7) quenching the reaction mixture with an acid to produce a biphasic solution containing the 1,4-dihydro-2H-3,1-benzoxazin-2-one in the organic solvent phase;
8) isolating the 1,4-dihydro-2H-3,1-benzoxazin-2-one from the organic phase.

The process for the preparation of a 1,4-dihydro-2H-3,1-benzoxazin-2-one as recited above, wherein the alkyl group of the alkyl chloroformate is defined as C₁-C₁₀-alkyl, which is optionally substituted with one, two or three substituents selected from the group consisting of: CF₃, C₃-C₇-cycloalkyl, CO₂C₁-C₆-alkyl, and NO_{2.} An embodiment of the alkyl choroformates useful in the process for the preparation of a 1,4-dihydro-2H-3,1-benzoxazin-2-one as recited above, are methyl or ethyl chloroformate.

The process for the preparation of a 1,4-dihydro-2H-3,1-benzoxazin-2-one as recited above, wherein the first base is defined as a solid or solution of KOH, NaOH, LiOH, K₂CO₃, Na₂CO₃, Li₂CO₃, KHCO₃, NaHCO₃, LiHCO₃, or a combination of said bases. The process for the preparation of a 1,4-dihydro-2H-3,1-benzoxazin-2-one as recited above, wherein the second base is defined as a solid or solution of KOC₁-C₆-alkyl, NaOC₁-C₆-alkyl, LiOC₁-C₆-alkyl, KC₁-C₆-alkyl, NaC₁-C₆-alkyl, LiC₁-C₆-alkyl, KHMDS, NaHMDS, LiHMDS, LDA or a combination of said bases.

The process for the preparation of a 1,4-dihydro-2H-3,1-benzoxazin-2-one as recited above, wherein the first organic solvent is selected form the group consisting of: methyl t-butyl ether, toluene, tetrahydrofuran, acetonitrile, or a combination of said solvents. The process for the preparation of a 1,4-dihydro-2H-3,1-benzoxazin-2-one as recited above, wherein the counter solvent is heptane. The process for the preparation of a 1,4-dihydro-2H-3,1-benzoxazin-2-one as recited above, wherein the second organic solvent is selected form the group consisting of: methyl t-butyl ether, toluene, tetrahydrofuran, C₁-C₆-alkanol, or a combination of said solvents.

The process for the preparation of a 1,4-dihydro-2H-3,1-benzoxazin-2-one as recited above, wherein the acid is selected form the group consisting of: HCl, HNO₃, H₂SO₄, and CH₃CO₂H.

The process for the preparation of 1,4-dihydro-2H-3,1-benzoxazin-2-one as recited above, comprising the additional step of: crystallizing the alkyl carbamate produced in step 4 from toluene-heptane or methyl t-butyl ether-heptane to produce the crystalline alkyl carbamate.

Another aspect of the invention is an alkyl carbamate of formula 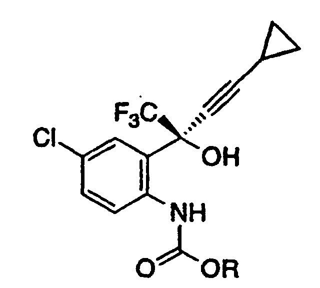 wherein R represents C₁-C₁₀-alkyl, which is optionally substituted with one, two or three substituents selected from the group consisting of: CF₃, C₃-C₇-cycloalkyl, CO₂C₁-C₆-alkyl, and NO₂.

As used herein, the term "alkyl" includes those alkyl groups of a designated number of carbon atoms of either a straight, branched, or cyclic configuration and optionally substituted with a substitutent selected from the group consisting of: CF₃, CO₂C₁-C₆-alkyl, C₃-C₇-cycloalkyl, and NO_{2.} Examples of "alkyl" include methyl, ethyl, propyl, isopropyl, butyl, sec-and tert-butyl, pentyl, hexyl, heptyl, cyclopropyl, cyclobutyl, cyclopentyl, cyclohexyl, cycloheptyl, norbornyl, and the like. The term "aryl" is defined as a phenyl or naphthyl ring which is optionally substituted one, two or three substitutents at any available carbon atoms selected from the group consisting of: halo (F, Cl, Br, I), CF₃, CO₂C₁-C₆-alkyl, and NO_{2.}

The term inert atmosphere is understood to be an atmosphere of argon or nitrogen, preferrably nitrogen.

Scheme 1 outlines the key steps in the synthesis of (-)-6-chloro-4-cyclopropylethynyl-4-trifluoromethyl-1,4-dihydro-2H-3,1-benzoxazin-2-one (DMP-266). The chiral addition step allows for the enantioselective addition of the cyclopropylacetylide across the trifluoromethylketone of **1.** The PMB-protected amino alcohol, **2,** produced is then deprotected to give the amino alcohol, **3.** The amino alcohol is then cyclized using a chloroformate and base to give DMP-266.

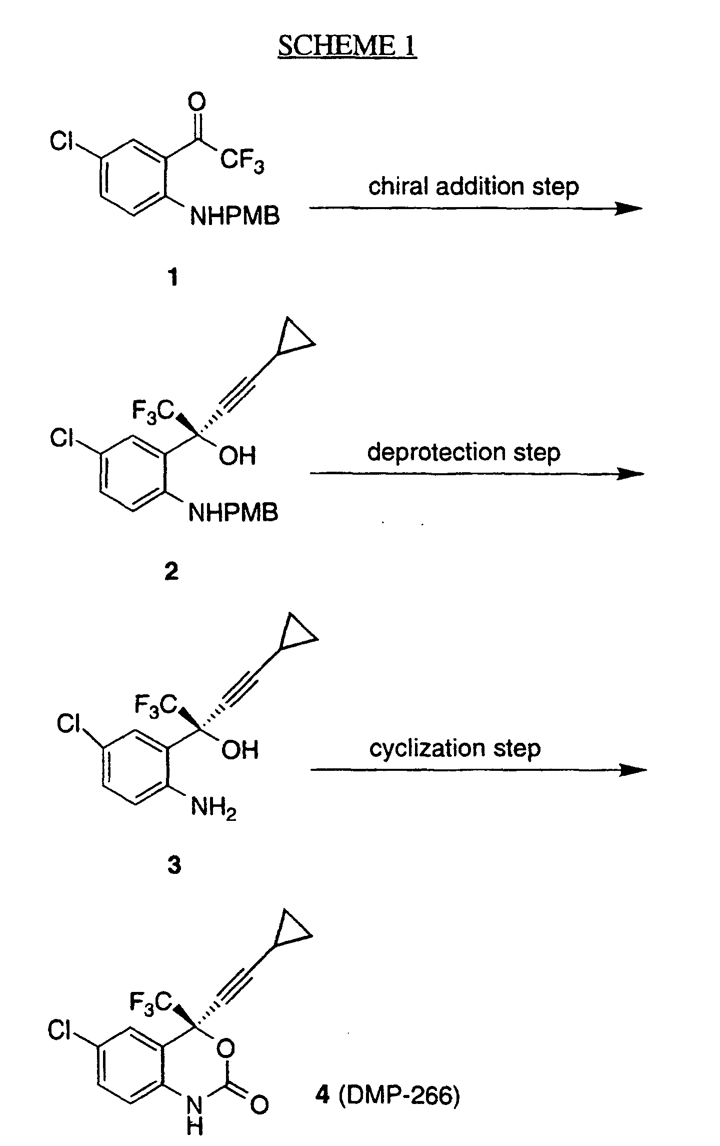

The cyclization of the amino alcohol, 3 to produce the 1,4-dihydro-2H-3,1-benzoxazin-2-one, 4 is outlined in Scheme 2 below. The reaction can be carried out as a one-step process, or alternatively a two step process with the potential isolation of the intermediate carbamate, **5** depending upon the chloroformate utilized. It has been demonstrated that the aryl chloroformates form less stable carbamates such that when they are treated with aqueous base they cyclize to the product, in a one-step process. The alkyl chloroformate, alternatively, provides an alkyl carbamate, a key intermediate capable of being isolated and purified prior to carrying out the cyclization step. Based upon the stability of the alkyl carbamates, a viable two step process for the preparation of DMP-266 has been developed which comprises the formation of the alkyl carbamate intermediate, 5 followed by the cyclization of the carbamate to give the desired product, **4**.

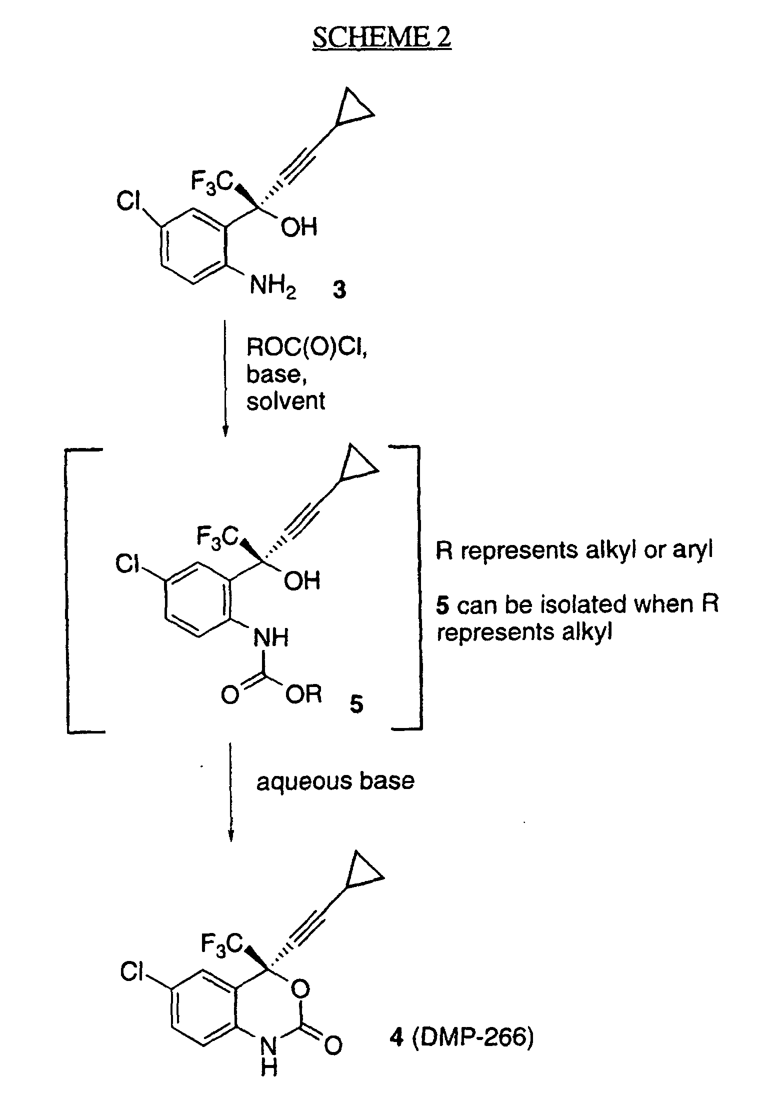

Table 1 lists the solvents and bases which can be used when running a one step process employing an aryl chloroformate. Additionally, combination of the bases listed below can and have been used, such as, bicarbonate and hydroxide, or bicarbonate and carbonate. The reaction can be run as a monophasic or biphasic reaction mixture. The base can be added at the start of the addition of the chloroformate or alternatively, after the aryl chloroformate addition is complete. The base is preferrably added at the start of the addition of the aryl chloroformate to quench the hydrogen chloride as it is produced.

**Table 1:**

| **One Step Process** | |
|---|---|
| solvent | base |
| ACN | carbonates(s) |
| | bicarbonates(s) |
| | hydroxides(s) |
| DMAC | carbonates(s) |
| | bicarbonates(s) |
| | hydroxides(s) |
| MTBE | carbonates(aq) |
| | bicarbonates(aq) |
| | hydroxides(aq) |
| NMP | carbonates(s) |
| | bicarbonates(s) |
| | hydroxides(s) |
| THF | carbonates(s) |
| | bicarbonates(s) |
| | hydroxides(s) |
| TOL | carbonates(aq) |
| | bicarbonates(aq) |
| | hydroxides(aq) |

Table 2 lists the solvents and bases which can be used when running two-step process employing an alkyl chloroformate. Step one of the two-step process, the formation of the alkylcarbamate, can be carried out as a monophasic or biphasic reaction mixture. Step two of the two-step process, ring closure of the alkyl carbamate, can be carried out in the solvetns noted using the listed bases.

**Table 2:**

| **Two Step Process** | | | |
|---|---|---|---|
| **Step 1:** Carbamate Formation | | **Step 2:** Ring Closure | |
| **1st solvent** | **1st base** | **2nd solvent** | **2nd base** |
| ACN† | carbonates(s) | alcohol | alkoxide |
| | bicarbonates(s) | THF | alkoxide |
| | hydroxides(s) | | alkyl alkaline metal |
| THF | carbonates(s) | | alkaline metal HMDS |
| | bicarbonates(s) | | LDA |
| | hydroxides(s) | MTBE | alkoxide |
| MTBE* | carbonates(aq) | | alkyl alkaline metal |
| | bicarbonates(aq) | | alkaline metal HMDS |
| | hydroxides(aq) | | LDA |
| TOL* | carbonates(aq) | TOL | alkoxide |
| | bicarbonates(aq) | | alkyl alkaline metal |
| | hydroxides(aq) | | alkaline metal HMDS |
| | | | LDA |

| | | | |
|---|---|---|---|
| † Use of acetonitrile as the first base may require an alternate isolation procedure, such as distilling the acetonitrile and cooling to effect crystallization of the carbamate, as opposed to distilling most of the first organic solvent, and then adding a counter solvent to crystallize the carbamate. | | | |
| * biphasic reaction mixtures. s refers to solid, when solid base is utilized in an anhydrous reaction mixture then the reaction is followed by a water quench. aq refers to an aqueous solution of the base. | | | |

Abbreviations for the solvents: acetonitrile (ACN); C₁-C₆-alcohol (alcohol); dimethylacetamide (DMAC); methyl t-butyl ether (MTBE); N-methylpyrrolidinone (NMP); tetrahydrofuran (THF); and toluene (TOL). Additionally, mixtures of the recited solvents may be used to optimize the reaction.
Abbreviations for the bases: lithium, sodium and potassium hydroxides; lithium, sodium and potassium carbonates; lithium, sodium and potassium bicarbonates; lithium, sodium and potassium alkyl (e.g. n-butyl lithium); lithium, sodium and potassium hexamethyldisilazide (e.g. LiHMDS); and lithium diisopropylamide (LDA).

The following examples are meant to be illustrative of the present invention. These examples are presented to exemplify the invention and are not to be construed as limiting the scope of the invention.

### EXAMPLE 1

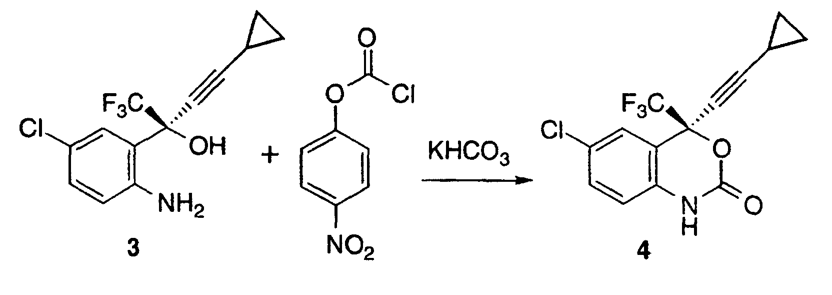

| | FW | g | mL | mmol | equiv |
|---|---|---|---|---|---|
| amino alcohol **12** | 289 | 7.0 | | 24.2 | 1 |
| 4-nitrophenyl | | | | | |
| chloroformate | 201.6 | 5.9 | | 29.3 | 1.2 |
| KHCO₃ | 100 | 7.26 | | 72.6 | 3 |
| K₂CO₃ | 138 | 10 | | 72.5 | 3 |
| H2O | | | 250 | | |
| THF | | | 100 | | |
| MTBE | | | 100 | | |
| EtOH | | | 45 | | |

To a three necked round bottom flask, equipped with a mechanical stirrer, nitrogen line, and thermocouple, was charged the solid amino alcohol **3,** THF (100 mL), and solid KHCO₃. The resulting mixture was cooled to +5°C and then solid 4-nitrophenyl chloroformate was added, in a single batch. This reaction is exothermic. A temperature rise of 4 degrees (final temp 9°C) was observed as the nitrophenyl chloroformate dissolved/reacted. The mixture was stirred at 20-25°C for two hours. A sample was taken from the batch and diluted with ACN and 5% NaHCO₃, to make a yellow, homogeneous solution. HPLC analysis at 220 nm showed nitrophenol (43%), **4** (49%), nitrophenyl carbonate (7%) and several low level (<0.3 %) impurities. Sampling the batch and diluting in this manner is important for reproducible data. If a sample of the batch is diluted with ACN and analyzed by HPLC, the major peak is that due to the nitrophenyl carbamate **5**. This is converted to **4** upon addition of aqueous base.

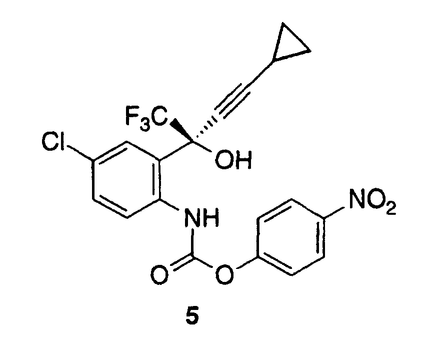

The reaction was quenched by addition of aq K₂CO₃ (10 g in 150 mL H₂O). The resulting two phase mixture was stirred vigorously at 25°C for two hours. MTBE (100 mL) was then added, the layers were separated, and the organic layer was washed with 5% aqueous K₂CO₃ (2x50 mL) and H₂O (2x50mL). The solvent was switched to ethanol (EtOH) or isopropanol (IPA), and the product crystallized from EtOH or IPA and water. See Examples 10 and 11.

### EXAMPLE 2

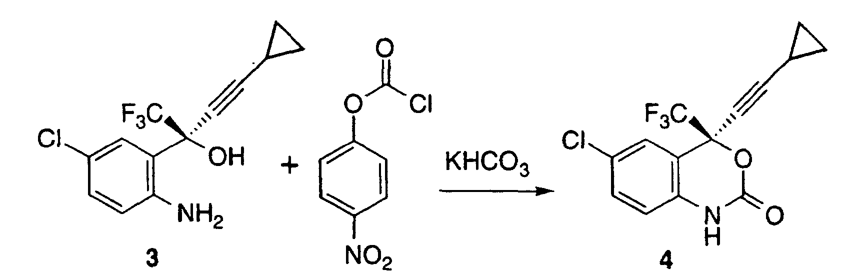

| | FW | g | mL | mmol | eq. |
|---|---|---|---|---|---|
| amino alcohol **12** | 289 | 7.0 | | 24.2 | 1 |
| 4-nitrophenylchloroformate | 201.6 | 5.9 | | 29.3 | 1.2 |
| K₂CO₃ | 138 | 20 | | 145.2 | 6 |
| H2O | | | 370 | | |
| MTBE | | | 100 | | |
| EtOH | | 45 | | | |

To a three necked round bottom flask, equipped with a mechanical stirrer, N₂ line, and thermocouple was charged the solid amino alcohol **12**, MTBE (100 mL), and aq K₂CO₃ (10 g in 120 mL H₂O, 3 equiv.). Solid 4-nitrophenyl chloroformate was added, in a single batch, at 25°C. The mixture was stirred at 20-25°C for two hours. At this point the two phase mixture was heated to 50°C for 3h, in order to effect conversion of nitrophenyl carbonate to nitrophenol. After cooling to 25°C, the layers were separated and the MTBE layer was extracted with aq K₂CO₃ (10 g in 150 mL H₂O, in two 75 mL portions) and then with water (2 X 50 ml). At this point the mixture was solvent switched to EtOH/IPA and crystallized as noted in Examples 10 and 11.

### EXAMPLE 3

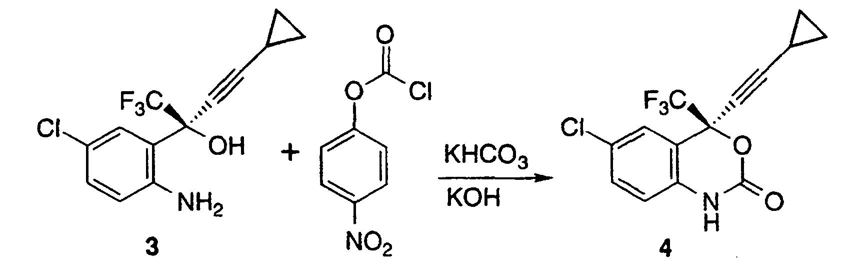

| | FW | g | mL | mmol | equiv |
|---|---|---|---|---|---|
| amino alcohol **12** | 289 | 100 | | 346 | 1 |
| 4-nitrophenylchloroformate | 201.6 | 80.2 | | 398 | 1.15 |
| KHCO₃ | 100 | 45 | | 450 | 1.3 |
| 2N KOH | 56 | | 346 | 692 | 2.0 |
| H₂O | | | 654 | | |
| MTBE | | | 500 | | |

To a three necked round bottom flask, equipped with a mechanical stirrer, nitrogen line, and thermocouple, was charged the solid amino alcohol **3,** MTBE (500 mL), and aqueous KHCO₃ (45 g in 654 mL H₂O). Solid 4-nitrophenyl chloroformate was added, in 4 batches, at 25°C. During the addition the solution pH was monitored. The pH was maintained between 8.5 and 4 during the reaction and ended up at 8.0. The mixture was stirred at 20-25°C for two hours. Aqueous KOH (2N) was added over 20 minutes, until the pH of the aqueous layer reached 11.0.

The layers were separated and the MTBE layer was washed with pH7 buffer (500 mL) and brine (500 mL). At this point the mixture was solvent switched to EtOH/IPA and crystallized as recited in Examples 10 and 11.

### EXAMPLE 4

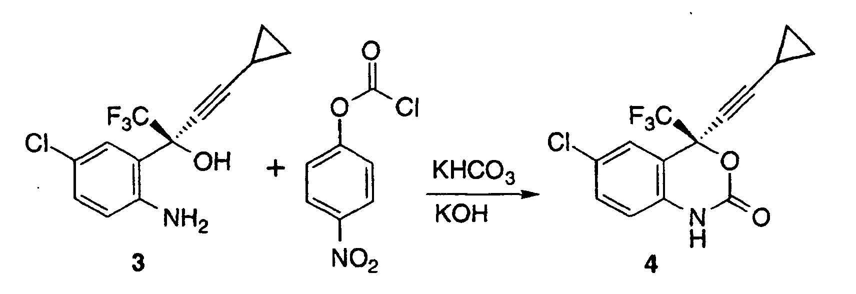

| | FW | g | mL | mmol | equiv |
|---|---|---|---|---|---|
| amino alcohol **3** | 289 | 100 | | 346 | 1 |
| 4-nitrophenylchloroformate | 201.6 | 73.2 | | 363 | 1.05 |
| KHCO₃ | 100 | 45 | | 450 | 1.3 |
| 2N KOH | 56 | | 346 | 692 | 2.0 |
| H₂O | | | 654 | | |
| MTBE | | | 500 | | |

To a three necked round bottom flask, equipped with a mechanical stirrer, nitrogen line, and thermocouple, was charged the solid amino alcohol **3**, MTBE (500 mL), and aqueous KHCO₃ (45 g in 654 mL H₂O). Solid 4-nitrophenyl chloroformate was added, in 4 batches, at 25°C. During the addition the solution pH was monitored. The pH was maintained between 8.5 and 4 during the reaction and ended up at 8.0. The mixture was stirred at 20-25°C for two hours. Aqueous KOH (2N) was added over 20 minutes, until the pH of the aqueous layer reached 11.0.

The layers were separated and 500 mL brine was added to the MTBE layer. 0.1 N Acetic acid was added until the pH was 6-7. The layers were separated and the organic phase was washed with brine (500 mL). At this point the mixture was solvent switched to EtOH/IPA and crystallized as recited in Examples 10 and 11.

### EXAMPLE 5

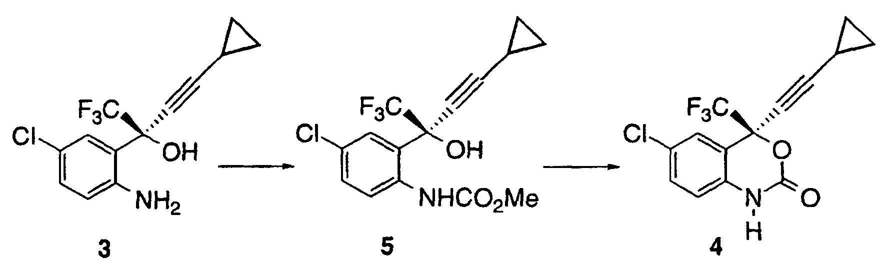

### Step A: Preparation of the carbamate, 5

| | MW | density | mmol | amount | equiv. |
|---|---|---|---|---|---|
| **12** | 289.7 | | 35.38 | 10.25 g | |
| KHCO₃ | 100.12 | | 70.76 | 7.08 g | 2 |
| ClCO₂Me | 94.50 | 1.223 | 70.76 | 5.46 mL | 2 |
| MTBE | | | | 100 mL | |
| water | | | | 100 mL | |
| brine | | | | 100 mL | |
| heptane | | | | 300 mL | |

A 500 mL 3-neck round bottom flask was equipped with an overhead stirrer, temperature probe, and nitrogen line. The amino alcohol 3 (35.38 mmol, 10.25 g) and MTBE (100 mL) were charged, forming a light slurry. The water (100 mL) was charged, followed by potassium bicarbonate (2 equ, 7.08 g). Methyl chloroformate (2 equiv., 5.46 mL) was charged via syringe, and the biphasic mixture was stirred vigorously at 20-25°C. Samples were assayed by HPLC until <0.5% amino alcohol 3 remained (approximately 8.5 hours). The layers were separated, and the organic layer was washed with brine (100 mL) and dried over magnesium sulfate. After filtration, a solvent switch (50-60°C under vacuum) was carried out into a MTBE - heptane mixture (approx. 5% MTBE by volume as measured by ¹H NMR) of 102 mL (10 mL/g starting material) total volume. The methyl carbamate **5** crystallized readily during the solvent switch. After aging the slurry at 20-25°C for approx. 30 min, the material was filtered. The solid was washed with the mother liquors, and then with one cake volume of heptane. The dry methyl carbamate **5** was isolated in 92% yield (11.32 g). 1-2% was lost to the mother liquors.

### Step B: Preparation of the ring closed compound, 4

| | MW | density | mmol | amount | equiv. |
|---|---|---|---|---|---|
| **5** | 347.72 | | 32.55 | 11.32 g | |
| 1M LiO^{t}Bu | | | 32.55 | 32.6 mL | 1 |
| MTBE | | | | 170 mL | |
| 0.5 N HCl | | | | 150 mL | |
| brine | | | | 150 mL | |

A 500 mL 3-neck round bottom flask was equipped with an overhead stirrer, temperature probe, and nitrogen line. The methyl carbamate **5** (32.55 mmol, 11.32 g) was dissolved in MTBE (170 mL). LiO^{t}Bu (1 equiv., 32.6 mL) was charged, and the reaction mixture immediately became a slurry. The slurry thinned with time and was a clear yellow solution within 30 min. The reaction mixture was aged at 20-25°C. The reaction was followed by HPLC. After 8 h, <1% methyl carbamate **5** remained. In approximately 16 hours, less than 0.3% methyl carbamate **5** remained. The reaction was quenched into 0.5 N HCl (150 mL). The layers were separated, and the organic layer was washed with brine (150 mL), dried over magnesium sulfate, and filtered. The HPLC assay yield was 96.0% of **4.** The material was solvent switched into EtOH in preparation for an EtOH-water crystallization. See Examples 10 and 11.

### EXAMPLE 6

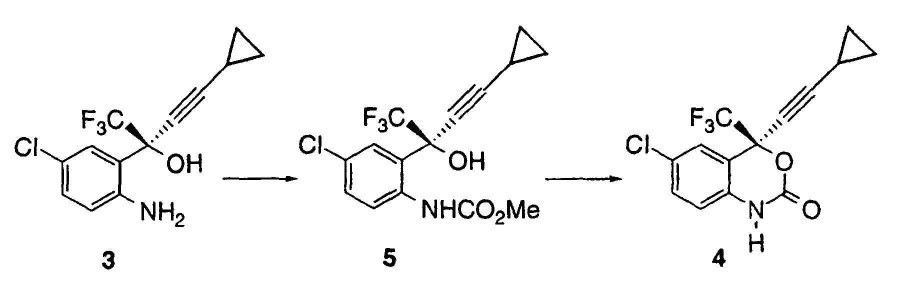

### Step A: Preparation of the carbamate, 5

Following the procedure as recited in Example 5, Step A except using toluene as the solvent, and isolation the carbamate from a mixture of toluene-heptane.

### Step B: Preparation of the ring closed compound, 4

Following the procedure as recited in Example 5, Step B except using toluene as the solvent, and isolating the product, **4** by crystallization from a mixture of toluene-heptane.

### EXAMPLE 7

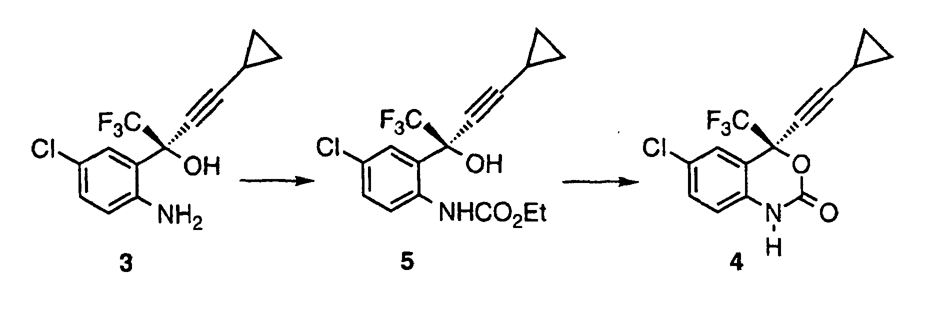

### Step A: Preparation of the carbamate, 5

| | MW | density | mmol | amount | equiv. |
|---|---|---|---|---|---|
| **3** | 289.7 | | 35.38 | 10.25 g | |
| KHCO₃ | 100.12 | | 70.76 | 7.08 g | 2 |
| ClCO₂Et | 108.52 | 1.135 | 70.76 | 6.76 mL | 2 |
| MTBE | | | | 100 mL | |
| water | | | | 100 mL | |
| brine | | | | 100 mL | |
| heptane | | | | 300 mL | |

A 500 mL 3-neck round bottom flask was equipped with an overhead stirrer, temperature probe, and nitrogen line. The amino alcohol 3 (35.38 mmol, 10.25 g) and MTBE (100 mL) were charged, forming a light slurry. The water (100 mL) was charged, followed by potassium bicarbonate (2 equ, 7.08 g). Ethyl chloroformate (2 equiv., 6.76 mL) was charged via syringe, and the biphasic mixture was stirred vigorously at 20-25°C. Samples were assayed by HPLC until <2% amino alcohol 3 remained (approximately 30 hours). The layers were separated, and the organic layer was washed with brine (100 mL) and dried over magnesium sulfate. After filtration, a solvent switch (50-60°C under vacuum) was carried out into a MTBE - heptane mixture (approx. 5% MTBE by volume as measured by ¹H NMR) of 102 mL (10 mL/g starting material) total volume. The ethyl carbamate **5** crystallized readily during the solvent switch. After aging the slurry at 20-25°C for approx. 30 min, the material was filtered. The solid was washed with the mother liquors, and then with one cake volume of heptane. The dry ethyl carbamate **5** was isolated in 92% yield (11.77 g). 1-2% was lost to the mother liquors.

### Step B: Preparation of the ring closed compound, 4

| | MW | density | mmol | amount | equiv. |
|---|---|---|---|---|---|
| **5** | 361.75 | | 32.55 | 11.77 g | |
| 1M LiO^{t}Bu | | | 32.55 | 32.6 mL | 1 |
| MTBE | | | | 170 mL | |
| 0.5 N HCl | | | | 150 mL | |
| brine | | | | 150 mL | |

A 500 mL 3-neck round bottom flask was equipped with an overhead stirrer, temperature probe, and nitrogen line. The ethyl carbamate **5** (32.55 mmol, 11.32 g) was dissolved in MTBE (170 mL). LiO^{t}Bu (1 equiv., 32.6 mL) was charged, and the reaction mixture immediately became a slurry. The slurry thinned with time and was a clear yellow solution within 30 min. The reaction mixture was aged at 20-25°C. The reaction was followed by HPLC. After 26 h, <1% ethyl carbamate 5 remained. The reaction was quenched into 0.5 N HCl (150 mL). The layers were separated, and the organic layer was washed with brine (150 mL), dried over magnesium sulfate, and filtered. The HPLC assay yield was 96.0% of **4.** The material was solvent switched into EtOH in preparation for an EtOH-water crystallization. See Examples 10 and 11.

### EXAMPLE 8

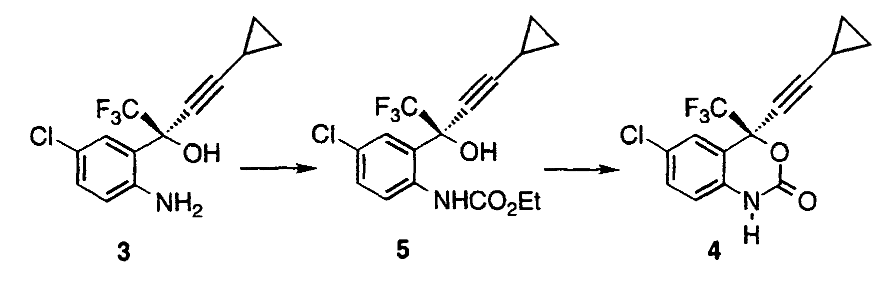

### Step A: Preparation of the carbamate, 5

Following the procedure as recited in Example 7, Step A except using toluene as the solvent, and isolation the carbamate from a mixture of toluene-heptane.

### Step B: Preparation of the ring closed compound, 4

Following the procedure as recited in Example 7, Step B except using toluene as the solvent, and isolating the product, **4** by crystallization from toluene-heptane.

### EXAMPLE 9

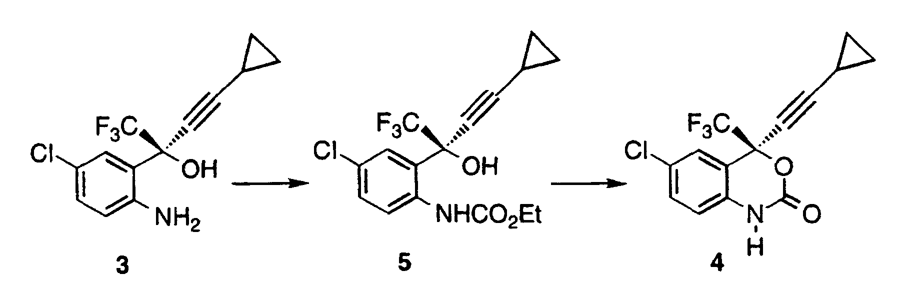

### Step A: Preparation of the carbamate, 5

Following the procedure as recited in Example 7, Step A.

### Step B: Preparation of the ring closed compound, 4

| | MW | density | mmol | amount | equiv. |
|---|---|---|---|---|---|
| **15** | 361.75 | | 32.55 | 11.77 g | |
| 2.5M nBuLi | | | 32.55 | 13.0 mL | 1 |
| MTBE | | | | 170 mL | |
| pH 7 buffer soln. | | | | 150 mL | |
| brine | | | | 150 mL | |

A 500 mL 3-neck round bottom flask was equipped with an overhead stirrer, temperature probe, and nitrogen line. The ethyl carbamate 5 (32.55 mmol, 11.32 g) was dissolved in MTBE (170 mL). nBuLi (1 equiv., 13.0 mL) was charged, and the reaction mixture was aged at 20-25°C. The reaction was followed by HPLC. After 30 h, <1% ethyl carbamate **5** remained. The reaction was quenched into pH 7 buffer solution (150 mL). The layers were separated, and the organic layer was washed with brine (150 mL), dried over magnesium sulfate, and filtered. The HPLC assay yield was 96.0% of **4**. The material was solvent switched into EtOH in preparation for an EtOH-water crystallization. See Examples 10 and 11.

### EXAMPLE 10

### Controlled Anti-Solvent Addition Crystallization Process

400 g. of DMP-266 starting material is dissolved in 2.400 L of ethanol. The solution is filtered to remove extraneous matter. 2.088 L of deionized (DI) water is added to the solution over 30 to 60 minutes. 20 g. of DMP-266 seed is added to the solution. The seed bed is aged for **1** hour. The use of Intermig agitators is preferred to mix the slurry. If required (by the presence of extremely long crystals or a thick slurry), the slurry is wet-milled for 15 - 60 seconds. 1.512 L of DI water is added to the slurry over 4 to 6 hours. If required (by the presence of extremely long crystals or a thick slurry), the slurry is wet-milled for 15 to about 60 seconds during the addition. The slurry is aged for 1 to 3 hours before being cooled to 10°C over 3 hours. The slurry is aged for 2 to 16 hours until the product concentration in the supernatant remains constant. The slurry is filtered to isolate a crystalline wet cake. The wet cake is washed with 1 to 2 bed volumes of 40 % ethanol in water and then twice with 2 L of DI water each. The washed wet cake is dried under vacuum at 50°C.

### EXAMPLE 11

### Semi-Continuous Heel Crystallization Process

400 g. of DMP-266 starting material is dissolved in 2.400 L of ethanol. A heel slurry is produced by mixing 20 g of DMP-266 in 0.3 L of 40 % (v/v) ethanol in water. The dissolved batch and 3.6 L of DI water are simultaneously charged to the heel slurry at constant rates over 6 hours to maintain a constant solvent composition in the crystallizer. Use of Intermig agitators during the crystallization is preferred. During this addition the slurry is wet-milled when the crystal lengths become excessively long or the slurry becomes too thick. The slurry is cooled to about 10°C over 3 hours. The slurry is aged for 2 to 16 hours until the product concentration in the supernatant remains constant. The slurry is filtered to isolate a crystalline wet cake. The wet cake is washed with 1 to 2 bed volumes of 40 % ethanol in water and then twice with 2 L of DI water each. The washed wet cake is dried under vacuum at 50°C.

## Claims

1. A process for the preparation of a 1,4-dihydro-2H-3,1-benzoxazin-2-one of the formula 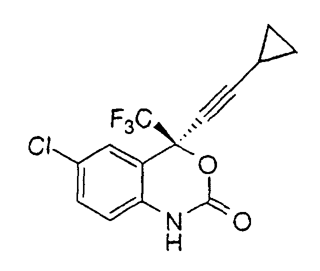 comprising the steps of:
1) adding an aryl chloroformate to a stirring mixture of an amino alcohol of formula 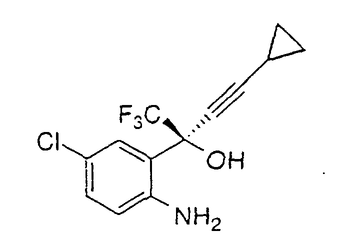 in an organic solvent with a base at a temperature of about 0°C to about 25°C under an inert atmosphere to produce a carbamate intermediate of formula 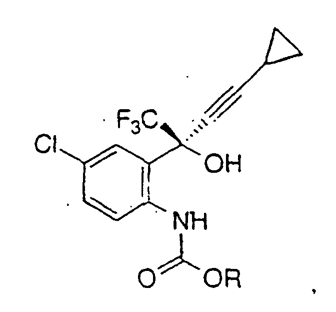 wherein R represents the aryl side chain of the chloroformate which is defined as phenyl or naphthyl, which is optionally substituted with one, two or three substituents selected from the group consisting of: halo (F, Cl, Br, I), CF₃, CO₂C₁-C₆-alkyl, and NO₂;
2) stirring the reaction mixture at about 20°C to about 25°C for about 1 to about 6 hours to complete the formation of the carbamate intermediate;
3) quenching the reaction with water or an aqueous base to produce a biphasic solution containing the 1,4-dihydro-2H-3,1-benzoxazin-2-one in the organic solvent phase;
4) stirring the biphasic mixture at about 20°C to about 50°C for about 1 to about 6 hours to complete the cyclization to the 1,4-dihydro-2H-3,1-benzoxazin-2-one; and
5) isolating the 1,4-dihydro-2H-3,1-benzoxazin-2-one from the organic phase.

2. The process for the preparation of a 1,4-dihydro-2H-3,1-benzoxazin-2-one as recited in Claim 1, wherein the base is defined as a solid or solution of KOH, NaOH, LiOH, K₂CO₃, Na₂CO₃, Li₂CO₃, KHCO₃, NaHCO₃, LiHCO₃, or a combination of said bases.

3. The process for the preparation of a 1,4-dihydro-2H-3,1-benzoxazin-2-one as recited in Claim 2, wherein the organic solvent is selected form the group consisting of: methyl t-butyl ether, toluene, tetrahydrofuran, acetonitrile, dimethylacetamide, N-methylpyrrolidinone, or a combination of said solvents.

4. The process for the preparation of a 1.4-dihydro-2H-3,1-benzoxazin-2-one as recited in Claim 3, wherein the aryl group of the aryl chloroformate is defined as phenyl chloroformate, in which the phenyl is optionally substituted with one, two or three substituents selected from the group consisting of: halo (F, Cl, Br, I), CF₃, and NO_{2.}

5. The process for the preparation of a 1,4-dihydro-2H-3,1-benzoxazin-2-one as recited in Claim 4, wherein the base is defined as a solid or solution of KOH, NaOH, K₂CO₃, Na₂CO₃, KHCO₃, NaHCO₃, or a combination of said bases.

6. The process for the preparation of a 1,4-dihydro-2H-3,1-benzoxazin-2-one as recited in Claim 5, wherein the aryl group of the aryl chloroformate is defined as 4-nitrophenyl chloroformate.

7. A process for the preparation of a 1,4-dihydro-2H-3,1-benzoxazin-2-one of the formula 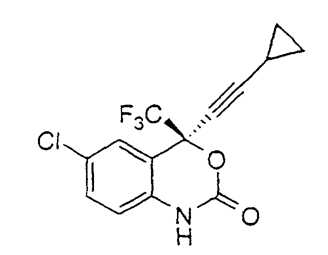 comprising the steps of:
1) adding 4-nitrophenyl chloroformate in batches to a stirring mixture of an amino alcohol of formula 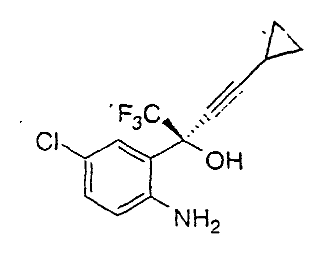 in methyl tert-butyl ether with an aqueous solution of KHCO₃ at a temperature of about 25°C under a nitrogen atmosphere maintaining a pH of between about 8.5 and 4 to produce a carbamate intermediate of formula 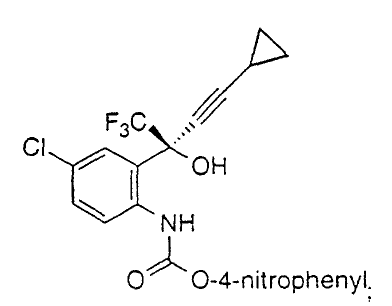
2) stirring the reaction mixture at about 20°C to about 25°C for about 2 hours to complete the formation of the carbamate intermediate;
3) quenching the reaction with an aqueous KOH to a pH of about 11 and adding water to produce a biphasic mixture containing the 1,4-dihydro-2H-3,1-benzoxazin-2-one in the organic solvent phase;
4) isolating the 1,4-dihydro-2H-3,1-benzoxazin-2-one from the organic phase.

8. A process for the preparation of a 1,4-dihydro-2H-3,1-benzoxazin-2-one of the formula 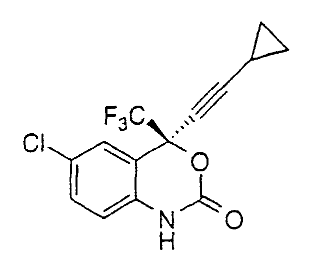 comprising the steps of:
1) adding an aryl chloroformate to a stirring mixture of an amino alcohol of formula 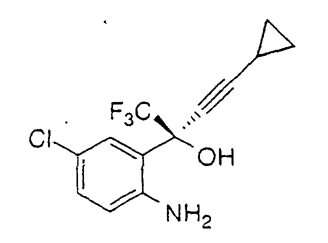 in an organic solvent at a temperature of about 0°C to about 25°C under an inert atmosphere to produce a carbamate intermediate of formula 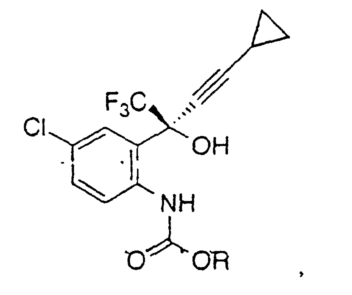 wherein R represents the aryl side chain of the chloroformate which is defined as phenyl or naphthyl, which is optionally substituted with one, two or three substituents selected from the group consisting of: halo (F, Cl, Br, I), CF₃, CO₂C₁-C₆-alkyl, and NO₂;
2) stirring the reaction mixture at about 20°C to about 25°C for about 1 to about 6 hours to complete the formation of the carbamate intermediate;
3) quenching the reaction with an aqueous base to produce a biphasic solution containing the 1,4-dihydro-2H-3,1-benzoxazin-2-one in the organic solvent phase;
4) stirring the biphasic mixture at about 20°C to about 50°C for about 1 to about 6 hours to complete the cyclization to the 1,4-dihydro-2H-3,1-benzoxazin-2-one; and
5) isolating the 1,4-dihydro-2H-3,1-benzoxazin-2-one from the organic phase.

9. The process for the preparation of a 1,4-dihydro-2H-3,1-benzoxazin-2-one as recited in Claim 8, wherein the base is defined as a solid or solution of KOH, NaOH, LiOH, K₂CO₃, Na₂CO₃, Li₂CO₃, KHCO₃, NaHCO₃, LiHCO₃, or a combination of said bases.

10. The process for the preparation of a 1,4-dihydro-2H-3,1-benzoxazin-2-one as recited in Claim 9, wherein the organic solvent is selected form the group consisting of: methyl t-butyl ether, toluene, or a combination of said solvents.

11. The process for the preparation of a 1,4-dihydro-2H-3,1-benzoxazin-2-one as recited in Claim 10, wherein the aryl group of the aryl chloroformate is defined as phenyl chloroformate, in which the phenyl is optionally substituted with one two or three substituents selected from the group consisting of: halo (F, Cl, Br, I), CF₃, and NO_{2.}

12. The process for the preparation of a 1,4-dihydro-2H-3,1-benzoxazin-2-one as recited in Claim 11, wherein the base is defined as a solid or solution of KOH, NaOH, K₂CO₃, Na₂CO₃, KHCO₃, NaHCO₃, or a combination of said bases.

13. The process for the preparation of a 1,4-dihydro-2H-3,1-benzoxazin-2-one as recited in Claim 12, wherein the aryl group of the aryl chloroformate is defined as 4-nitrophenyl chloroformate.

14. A process for the preparation of a 1,4-dihydro-2H-3,1-benzoxazin-2-one of the formula 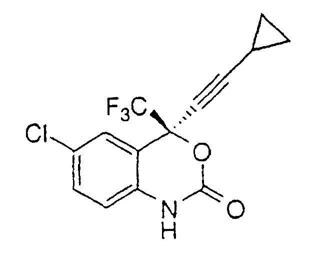 comprising the steps of:
1) adding an alkyl chloroformate to a stirring mixture of an amino alcohol of formula 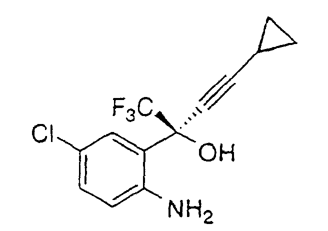 in a first organic solvent with a first base at a temperature of about 0°C to about 25°C under an inert atmosphere to produce a carbamate intermediate of formula 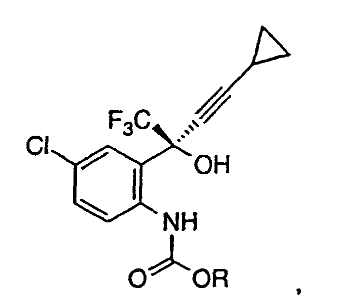 wherein R represents the alkyl side chain of the chloroformate which is denned as C₁-C₁₀-alkyl, which is optionally substituted with one, two or three substituents selected from the group conisisting of: CF₃, C₃-C₇-cycloalkyl, CO₂C₁-C₆-alkyl, and NO₂;
2) stirring the reaction mixture at about 20°C to about 25°C for about 1 to about 30 hours to complete the formation of the carbamate intermediate;
3) isolating the organic phase containing the alkyl carbamate;
4) distilling about 90% to about 95% of the first organic solvent in vacuum and adding a counter solvent to isolate the solid alkyl carbamate;
5) adding a second organic solvent to the solid alkyl carbamate to form an alkyl carbamate solution;
6) reacting the alkyl carbamate solution with a second base at a temperature range of about 20°C to about 25°C for about 2 hours to about 30 hours to produce the 1,4-dihydro-2H-3,1-benzoxazin-2-one;
7) quenching the reaction mixture with an acid to produce a biphasic solution containing the 1,4-dihydro-2H-3,1-benzoxazin-2-one in the organic solvent phase;
8) isolating the 1,4-dihydro-2H-3,1-benzoxazin-2-one from the organic phase.

15. The process for the preparation of a 1,4-dihydro-2H-3,1-benzoxazin-2-one as recited in Claim 14, wherein the first base is defined as a solid or solution of KOH, NaOH, LiOH, K₂CO₃, Na₂CO₃, Li₂CO₃, KHCO₃, NaHCO₃, LiHCO₃, or a combination of said bases.

16. The process for the preparation of a 1,4-dihydro-2H-3,1-benzoxazin-2-one as recited in Claim 15, wherein the first organic solvent is selected form the group consisting of: methyl t-butyl ether, toluene, tetrahydrofuran, acetonitrile, or a combination of said solvents.

17. The process for the preparation of a 1,4-dihydro-2H-3,1-benzoxazin-2-one as recited in Claim 16, wherein the counter solvent is heptane.

18. The process for the preparation of a 1,4-dihydro-2H-3,1-benzoxazin-2-one as recited in Claim 17, wherein the second organic solvent is selected form the group consisting of: methyl t-butyl ether, toluene, tetrahydrofuran, C₁-C₆-alkanol, or a combination of said solvents.

19. The process for the preparation of a 1,4-dihydro-2H-3,1-benzoxazin-2-one as recited in Claim 18, wherein the second base is defined as a solid or solution of KOC₁-C₆-alkyl, NaOC₁-C₆-alkyl, LiOC₁-C₆-alkyl, KC₁-C₆-alkyl, NaC₁-C₆-alkyl, LiC₁-C₆-alkyl, KHMDS, NaHMDS, LiHMDS, LDA or a combination of said bases.

20. The process for the preparation of a 1,4-dihydro-2H-3,1-benzoxazin-2-one as recited in Claim 19, wherein the acid is selected form the group consisting of: HCl, HNO₃, H₂SO₄, and CH₃CO₂H.

21. The process for the preparation of 1,4-dihydro-2H-3,1-benzoxazin-2-one as recited in Claim 20, comprising the additional step of: crystallizing.the alkyl carbamate produced in step 4 from toluene-heptane or methyl t-butyl ether-heptane to produce the desired crystalline alkyl carbamate.

22. The process for the preparation of a 1,4-dihydro-2H-3,1-benzoxazin-2-one as recited in Claim 21, wherein the alkyl chloroformate is defined as methyl or ethyl chloroformate.

23. The process for the preparation of a 1,4-dihydro-2H-3,1-benzoxazin-2-one as recited in Claim 22, wherein the organic solvent (or solvent mixture) is selected form the group consisting of: methyl t-butyl ether; methyl t-butyl ether and tetrahydrofuran; and methyl t-butyl ether and ethanol.

24. The process for the preparation of a 1,4-dihydro-2H-3,1-benzoxazin-2-one as recited in Claim 23, wherein the base is defined as a solid or solution of KHCO₃ and KOH, KHCO₃ and K₂CO₃, or KHCO₃, and LiO^{t}Bu.

25. An alkyl carbamate of formula 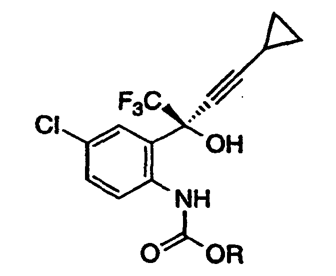 wherein R represents C₁-C₁₀-alkyl, which is optionally substituted with one, two or three substituents selected from the group consisting of: CF₃, C₃-C₇-cycloalkyl, CO₂C₁-C₆-alkyl, and NO_{2.}

## Patentansprüche

1. Ein Verfahren zur Herstellung eines 1,4-Dihydro-2H-3,1-benzoxazin-2-ons der Formel 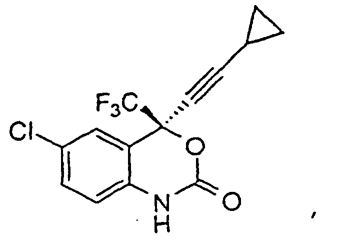 umfassend die Schritte:
1) Zugabe eines Arylchlorformiats zu einer gerührten Mischung eines Aminoalkohols der Formel 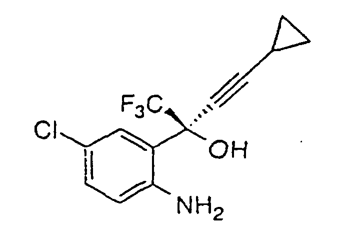 in einem organischen Lösungsmittel mit einer Base bei einer Temperatur von etwa 0°C bis etwa 25°C unter einer inerten Atmosphäre, um ein Carbamat-Zwischenprodukt der Formel 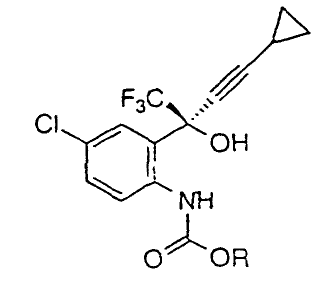 zu erzeugen, wobei R die Arylseitenkette des Chlorformiats bedeutet, die als Phenyl oder Naphthyl definiert ist, das gegebenenfalls substituiert ist mit ein, zwei oder drei Substituenten, ausgewählt aus der Gruppe, bestehend aus: Halogen (F, Cl, Br, I), CF₃, CO₂C₁-C₆-Alkyl und NO₂,
2) Rühren der Reaktionsmischung bei etwa 20°C bis etwa 25°C etwa 1 bis etwa 6 Stunden lang, um die Bildung des Carbamat-Zwischenprodukts zu vervollständigen,
3) Quenchen der Reaktion mit Wasser oder einer wäßrigen Base, um eine zweiphasige Lösung zu erzeugen, die das 1,4-Dihydro-2H-3,1-benzoxazin-2-on in der organischen Lösungsmittelphase enthält,
4) Rühren der zweiphasigen Mischung bei etwa 20°C bis etwa 50°C etwa 1 bis etwa 6 Stunden lang, um die Cyclisierung zum 1,4-Dihydro-2H-3,1-benzoxazin-2-on zu vervollständigen, und
5) Isolieren des 1,4-Dihydro-2H-3,1-benzoxazin-2-ons aus der organischen Phase.

2. Das wie in Anspruch 1 wiedergegebene Verfahren zur Herstellung eines 1,4-Dihydro-2H-3,1-benzoxazin-2-ons, bei dem die Base definiert ist als ein Feststoff oder eine Lösung aus KOH, NaOH, LiOH, K₂CO₃, Na₂CO₃, Li₂CO₃, KHCO₃, NaHCO₃, LiHCO₃ oder eine Kombination der genannten Basen.

3. Das wie in Anspruch 2 wiedergegebene Verfahren zur Herstellung eines 1,4-Dihydro-2H-3,1-benzoxazin-2-ons, bei dem das organische Lösungsmittel ausgewählt ist aus der Gruppe, bestehend aus: Methyl-t-butylether, Toluol, Tetrahydrofuran, Acetonitril, Dimethylacetamid, N-Methylpyrrolidinon oder einer Kombination aus den genannten Lösungsmitteln.

4. Das wie in Anspruch 3 wiedergegebene Verfahren zur Herstellung eines 1,4-Dihydro-2H-3,1-benzoxazin-2-ons, bei dem die Arylgruppe des Arylchlorformiats definiert ist als Phenylchlorformiat, wobei das Phenyl gegebenenfalls substituiert ist mit ein, zwei oder drei Substituenten, ausgewählt aus der Gruppe, bestehend aus: Halogen (F, Cl, Br, I), CF₃ und NO₂.

5. Das wie in Anspruch 4 wiedergegebene Verfahren zur Herstellung eines 1,4-Dihydro-2H-3,1-benzoxazin-2-ons, bei dem die Base definiert ist als ein Feststoff oder eine Lösung aus KOH, NaOH, K₂CO₃, Na₂CO₃, KHCO₃, NaHCO₃ oder eine Kombination aus diesen Basen.

6. Das wie in Anspruch 5 wiedergegebene Verfahren zur Herstellung eines 1,4-Dihydro-2H-3,1-benzoxazin-2-ons, bei dem die Arylgruppe des Arylchlorformiats als 4-Nitrophenylchlorformiat definiert ist.

7. Ein Verfahren zur Herstellung eines 1,4-Dihydro-2H-3,1-benzoxazin-2-ons der Formel 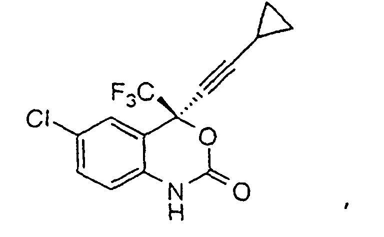 umfassend die Schritte:
1) Zugabe von 4-Nitrophenylchlorformiat chargenweise zu einer gerührten Mischung eines Aminoalkohols der Formel 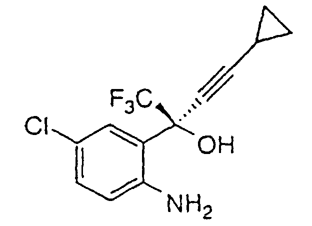 in Methyl-tert.-butylether mit einer wäßrigen KHCO₃-Lösung bei einer Temperatur von etwa 25°C unter einer Stickstoffatmosphäre, wobei ein pH-Wert von etwa 8,5 bis 4 aufrechterhalten wird, um ein Carbamat-Zwischenprodukt der Formel 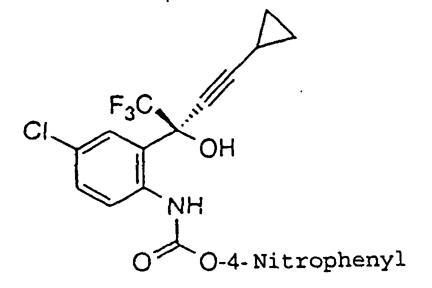 zu erzeugen,
2) Rühren der Reaktionsmischung bei etwa 20°C bis etwa 25°C etwa 2 Stunden lang, um die Bildung des Carbamat-Zwischenprodukts zu vervollständigen,
3) Quenchen der Reaktion mit wäßriger KOH bis zu einem pH-Wert von etwa 11 und Zugabe von Wasser, um eine zweiphasige Mischung zu erzeugen, die das 1,4-Dihydro-2H-3,1-benzoxazin-2-on in der organischen Lösungsmittelphase enthält,
4) Isolieren des 1,4-Dihydro-2H-3,1-benzoxazin-2-ons aus der organischen Phase.

8. Ein Verfahren zur Herstellung eines 1,4-Dihydro-2H-3,1-benzoxazin-2-ons der Formel 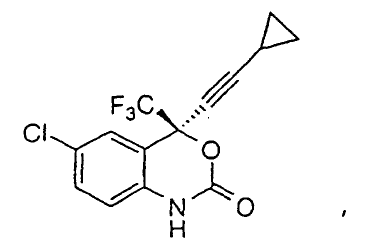 umfassend die Schritte:
1) Zugabe eines Arylchlorformiats zu einer gerührten Mischung eines Aminoalkohols der Formel 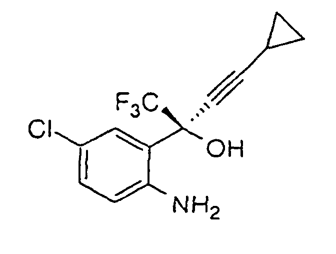 in einem organischen Lösungsmittel bei einer Temperatur von etwa 0°C bis etwa 25°C unter einer inerten Atmosphäre, um ein Carbamat-Zwischenprodukt der Formel 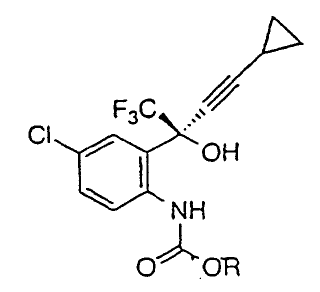 zu erzeugen, wobei R die Arylseitenkette des Chlorformiats bedeutet, die als Phenyl oder Naphthyl definiert ist, das gegebenenfalls substituiert ist mit ein, zwei oder drei Substituenten, ausgewählt aus der Gruppe, bestehend aus: Halogen (F, Cl, Br, I), CF₃, CO₂C₁-C₆-Alkyl und NO₂,
2) Rühren der Reaktionsmischung bei etwa 20°C bis etwa 25°C etwa 1 bis etwa 6 Stunden lang, um die Bildung des Carbamat-Zwischenprodukts zu vervollständigen,
3) Quenchen der Reaktion mit einer wäßrigen Base, um eine zweiphasige Lösung zu erzeugen, die das 1,4-Dihydro-2H-3,1-benzoxazin-2-on in der organischen Lösungsmittelphase enthält,
4) Rühren der zweiphasigen Mischung bei etwa 20°C bis etwa 50°C etwa 1 bis etwa 6 Stunden lang, um die Cyclisierung zum 1,4-Dihydro-2H-3,1-benzoxazin-2-on zu vervollständigen, und
5) Isolieren des 1,4-Dihydro-2H-3,1-benzoxazin-2-ons aus der organischen Phase.

9. Das wie in Anspruch 8 wiedergegebene Verfahren zur Herstellung eines 1,4-Dihydro-2H-3,1-benzoxazin-2-ons, bei dem die Base definiert ist als ein Feststoff oder eine Lösung aus KOH, NaOH, LiOH, K₂CO₃, Na₂CO₃, Li₂CO₃, KHCO₃, NaHCO₃, LiHCO₃ oder eine Kombination der genannten Basen.

10. Das wie in Anspruch 9 wiedergegebene Verfahren zur Herstellung eines 1,4-Dihydro-2H-3,1-benzoxazin-2-ons, bei dem das organische Lösungsmittel ausgewählt ist aus der Gruppe, bestehend aus: Methyl-t-butylether, Toluol oder einer Kombination aus den genannten Lösungsmitteln.

11. Das wie in Anspruch 10 wiedergegebene Verfahren zur Herstellung eines 1,4-Dihydro-2H-3,1-benzoxazin-2-ons, bei dem die Arylgruppe des Arylchlorformiats definiert ist als Phenylchlorformiat, wobei das Phenyl gegebenenfalls substituiert ist mit ein, zwei oder drei Substituenten, ausgewählt aus der Gruppe, bestehend aus: Halogen (F, Cl, Br, I), CF₃ und NO₂.

12. Das wie in Anspruch 11 wiedergegebene Verfahren zur Herstellung eines 1,4-Dihydro-2H-3,1-benzoxazin-2-ons, bei dem die Base definiert ist als ein Feststoff oder eine Lösung aus KOH, NaOH, K₂CO₃, Na₂CO₃, KHCO₃, NaHCO₃ oder eine Kombination aus diesen Basen.

13. Das wie in Anspruch 12 wiedergegebene Verfahren zur Herstellung eines 1,4-Dihydro-2H-3,1-benzoxazin-2-ons, bei dem die Arylgruppe des Arylchlorformiats als 4-Nitrophenylchlorformiat definiert ist.

14. Ein Verfahren zur Herstellung eines 1,4-Dihydro-2H-3,1-benzoxazin-2-ons der Formel 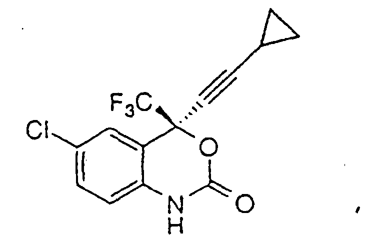 umfassend die Schritte:
1) Zugabe eines Arylchlorformiats zu einer gerührten Mischung eines Aminoalkohols der Formel 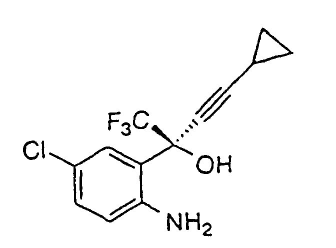 in einem ersten organischen Lösungsmittel mit einer ersten Base bei einer Temperatur von etwa 0°C bis etwa 25°C unter einer inerten Atmosphäre, um ein Carbamat-Zwischenprodukt der Formel 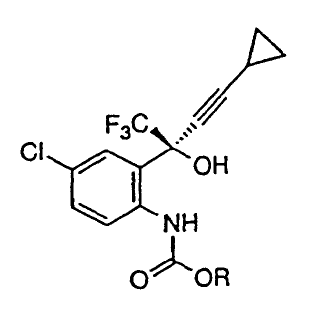 zu erzeugen, wobei R die Alkylseitenkette des Chlorformiats bedeutet, die als C₁-C₁₀-Alkyl definiert ist, gegebenenfalls substituiert mit ein, zwei oder drei Substituenten, ausgewählt aus der Gruppe, bestehend aus: CF₃, C₃-C₇-Cycloalkyl, CO₂C₁-C₆-Alkyl und NO₂,
2) Rühren der Reaktionsmischung bei etwa 20°C bis etwa 25°C etwa 1 bis etwa 30 Stunden lang, um die Bildung des Carbamat-Zwischenprodukts zu vervollständigen,
3) Isolieren der organischen Phase, die das Alkylcarbamat enthält,
4) Abdestillieren von etwa 90% bis etwa 95% des ersten organischen Lösungsmittels im Vakuum und Zugabe eines Gegenlösungsmittels, um das feste Alkylcarbamat zu isolieren,
5) Zugabe eines zweiten organischen Lösungsmittel zu dem festen Alkylcarbamat, um eine Alkylcarbamatlösung zu bilden,
6) Umsetzung der Alkylcarbamatlösung mit einer zweiten Base bei einem Temperaturbereich von etwa 20°C bis etwa 25'C etwa 2 Stunden bis etwa 30 Stunden lang, um das 1,4-Dihydro-2H-3,1-benzoxazin-2-on zu erzeugen,
7) Quenchen der Reaktionsmischung mit einer Säure, um eine zweiphasige Lösung zu erzeugen, die das 1,4-Dihydro-2H-3,1-benzoxazin-2-on in der organischen Lösungsmittelphase enthält,
8) Isolieren des 1,4-Dihydro-2H-3,1-benzoxazin-2-ons aus der organischen Phase.

15. Das wie in Anspruch 14 wiedergegebene Verfahren zur Herstellung eines 1,4-Dihydro-2H-3,1-benzoxazin-2-ons, bei dem die erste Base definiert ist als ein Feststoff oder eine Lösung aus KOH, NaOH, LiOH, K₂CO₃, Na₂CO₃, Li₂CO₃, KHCO₃, NaHCO₃, LiHCO₃ oder eine Kombination der genannten Basen.

16. Das wie in Anspruch 15 wiedergegebene Verfahren zur Herstellung eines 1,4-Dihydro-2H-3,1-benzoxazin-2-ons, bei dem das erste organische Lösungsmittel ausgewählt ist aus der Gruppe, bestehend aus: Methyl-t-butylether, Toluol, Tetrahydrofuran, Acetonitril oder einer Kombination aus den genannten Lösungsmitteln.

17. Das wie in Anspruch 16 wiedergegebene Verfahren zur Herstellung eines 1,4-Dihydro-2H-3,1-benzoxazin-2-ons, bei dem das Gegenlösungsmittel Heptan ist.

18. Das wie in Anspruch 17 wiedergegebene Verfahren zur Herstellung eines 1,4-Dihydro-2H-3,1-benzoxazin-2-ons, bei dem das zweite organische Lösungsmittel ausgewählt ist aus der Gruppe, bestehend aus: Methyl-t-butylether, Toluol, Tetrahydrofuran, C₁-C₆-Alkanol oder einer Kombination aus den genannten Lösungsmitteln.

19. Das wie in Anspruch 18 wiedergegebene Verfahren zur Herstellung eines 1,4-Dihydro-2H-3,1-benzoxazin-2-ons, bei dem die zweite Base definiert ist als ein Feststoff oder eine Lösung aus KOC₁-C₆-Alkyl, NaOC₁-C₆-Alkyl, LiOC₁-C₆-Alkyl, KC₁-C₆-Alkyl, NaC₁-C₆₋Alkyl, LiC₁-C₆-Alkyl, KHMDS, NaHMDS, LiHMDS, LDA oder eine Kombination aus den genannten Basen.

20. Das wie in Anspruch 19 wiedergegebene Verfahren zur Herstellung eines 1,4-Dihydro-2H-3,1-benzoxazin-2-ons, bei dem die Säure ausgewählt ist aus der Gruppe, bestehend aus: HCl, HNO₃, H₂SO₄ und CH₃CO₂H.

21. Das wie in Anspruch 20 wiedergegebene Verfahren zur Herstellung eines 1,4-Dihydro-2H-3,1-benzoxazin-2-ons, umfassend den zusätzlichen Schritt: Umkristallisation des in Schritt 4 erzeugten Alkylcarbamats aus Toluol-Heptan oder Methyl-t-butylether-Heptan, um das erwünschte kristalline Alkylcarbamat zu erzeugen.

22. Das wie in Anspruch 21 wiedergegebene Verfahren zur Herstellung eines 1,4-Dihydro-2H-3,1-benzoxazin-2-ons, bei dem das Alkylchlorformiat definiert ist als Methyl- oder Ethylchlorformiat.

23. Das wie in Anspruch 22 wiedergegebene Verfahren zur Herstellung eines 1,4-Dihydro-2H-3,1-benzoxazin-2-ons, bei dem das organische Lösungsmittel (oder die Lösungsmittelmischung) ausgewählt ist aus der Gruppe, bestehend aus: Methyl-t-butylether, Methyl-t-butylether und Tetrahydrofuran, und Methyl-t-butylether und Ethanol.

24. Das wie in Anspruch 23 wiedergegebene Verfahren zur Herstellung eines 1,4-Dihydro-2H-3,1-benzoxazin-2-ons, bei dem die Base definiert ist als ein Feststoff oder eine Lösung aus KHCO₃ und KOH, KHCO₃ und K₂CO₃, oder KHCO₃ und LiO^{t}Bu.

25. Ein Alkylcarbamat der Formel 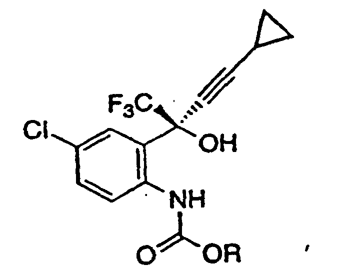 wobei R C₁-C₁₀-Alkyl bedeutet, das gegebenenfalls substituiert ist mit ein, zwei oder drei Substituenten, ausgewählt aus der Gruppe, bestehend aus: CF₃, C₃-C₇-Cycloalkyl, CO₂C₁-C₆-Alkyl und NO₂.

## Revendications

1. Procédé de préparation d'une 1,4-dihydro-2H-3,1-benzoxazin-2-one de formule 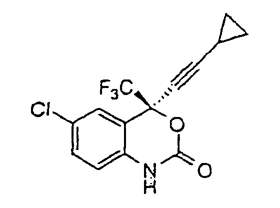 comprenant les étapes :
1) addition d'un chloroformate d'aryle à un mélange en agitation d'un aminoalcool de formule 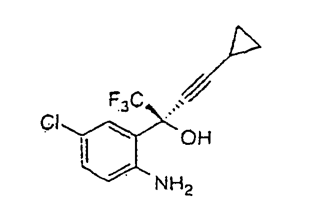 dans un solvant organique avec une base, à une température allant d'environ 0 à environ 25 °C, sous atmosphère inerte, afin de produire un carbamate intermédiaire de formule 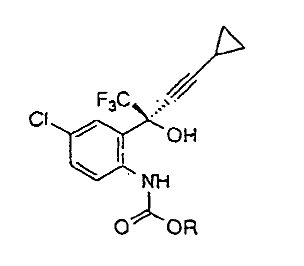 dans lequel R représente la chaîne latérale aryle du chloroformate définie comme un phényle ou un naphtyle, pouvant être mono-, di- ou trisubstitué par des substituants choisis dans le groupe consistant en : halogènes (F, Cl, Br, I), CF₃, alkoxycarbonyle en C₁ à C₆ et NO₂ ;
2) agitation du mélange réactionnel à une température allant d'environ 20 à environ 25 °C pendant environ 1 à environ 6 heures afin d'achever la formation du carbamate intermédiaire ;
3) refroidissement du milieu réactionnel à l'aide d'eau ou d'une base aqueuse afin d'obtenir une solution biphasique contenant la 1,4-dihydro-2H-3,1-benzoxazin-2-one en phase organique ;
4) agitation du mélange biphasique à une température allant d'environ 20 à environ 50 °C pendant environ 1 à environ 6 heures afin d'achever la cyclisation de la 1,4-dihydro-2H-3,1-benzoxazin-2-one ; et
5) isolation de la 1,4-dihydro-2H-3,1-benzoxazin-2-one de la phase organique.

2. Procédé de préparation d'une 1,4-dihydro-2H-3,1-benzoxazin-2-one selon la revendication 1, dans lequel la base est définie comme un solide ou une solution de KOH, NaOH, LiOH, K₂CO₃, Na₂CO₃, Li₂CO₃, KHCO₃, NaHCO₃, LiHCO₃ ou un mélange desdites bases.

3. Procédé de préparation d'une 1,4-dihydro-2H-3,1-benzoxazin-2-one selon la revendication 2, dans lequel le solvant organique est choisi dans le groupe consistant en : oxyde de tert-butyle et de méthyle, toluène, tétrahydrofuranne, acétonitrile, diméthylacétamide, N-méthylpyrrolidinone ou un mélange desdits solvants.

4. Procédé de préparation d'une 1,4-dihydro-2H-3,1-benzoxazin-2-one selon la revendication 3, dans lequel le groupe aryle du chloroformate d'aryle est défini comme le chloroformate de phényle, dans lequel le phényle peut être mono-, di- ou trisubstitué par des substituants choisis dans le groupe consistant en : halogènes (F, Cl, Br, I), CF₃ et NO₂.

5. Procédé de préparation d'une 1,4-dihydro-2H-3,1-benzoxazin-2-one selon la revendication 4, dans lequel la base est définie comme un solide ou une solution de KOH, NaOH, K₂CO₃, Na₂CO₃, KHCO₃, NaHCO₃ ou un mélange desdites bases.

6. Procédé de préparation d'une 1,4-dihydro-2H-3,1-benzoxazin-2-one selon la revendication 5, dans lequel le groupe aryle du chloroformate d'aryle est défini comme le 4-nitrophénylchloroformate.

7. Procédé de préparation d'une 1,4-dihydro-2H-3,1-benzoxazin-2-one de formule 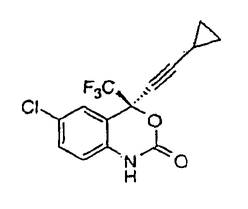 comprenant les étapes :
1) addition discontinue du 4-nitrophénylchloroformate à un mélange en agitation d'un aminoalcool de formule 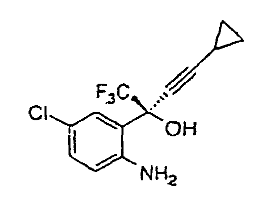 dans de l'oxyde de tert-butyle et de méthyle avec une solution aqueuse de KHCO_{3,} à une température d'environ 25 °C, sous azote, en maintenant un pH compris entre environ 8,5 et 4 afin de produire le carbamate intermédiaire de formule 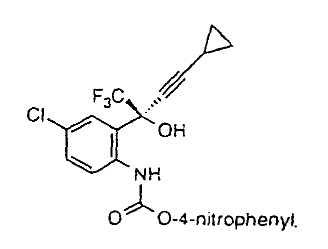
2) agitation du mélange réactionnel à une température allant d'environ 20 à environ 25°C pendant environ 2 heures afin d'achever la formation du carbamate intermédiaire ;
3) refroidissement du milieu réactionnel à l'aide d'une solution aqueuse de KOH à un pH d'environ 11 et addition d'eau afin d'obtenir un mélange biphasique contenant la 1,4-dihydro-2H-3,1-benzoxazin-2-one dans la phase organique ;
4) isolation de la 1,4-dihydro-2H-3,1-benzoxazin-2-one de la phase organique.

8. Procédé de préparation d'une 1,4-dihydro-2H-3,1-benzoxazin-2-one de formule 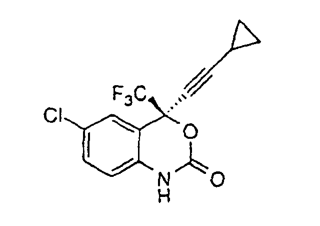 comprenant les étapes :
1) addition d'un chloroformate d'aryle à un mélange en agitation d'un aminoalcool de formule 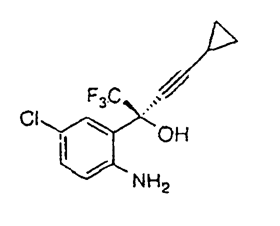 dans un solvant organique, à une température allant d'environ 0 à environ 25 °C, sous atmosphère inerte, afin de produire un carbamate intermédiaire de formule 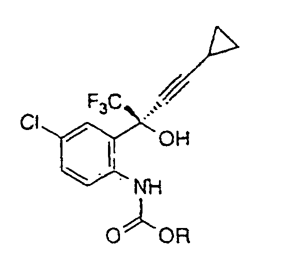 dans lequel R représente la chaîne latérale aryle du chloroformate définie comme un phényle ou un naphtyle, pouvant être mono-, di- ou trisubstitué par des substituants choisis dans le groupe consistant en : halogènes (F, Cl, Br, I), CF₃, alkoxycarbonyle en C₁ à C₆ et NO₂;
2) agitation du mélange réactionnel à une température allant d'environ 20 à environ 25°C pendant environ 1 à environ 6 heures afin d'achever la formation du carbamate intermédiaire ;
3) refroidissement du milieu réactionnel à l'aide d'une base aqueuse afin d'obtenir une solution biphasique contenant la 1,4-dihydro-2H-3,1-benzoxazin-2-one en phase organique ;
4) agitation du mélange biphasique à une température allant d'environ 20 à environ 50 °C pendant environ 1 à environ 6 heures afin d'achever la cyclisation de la 1,4-dihydro-2H-3,1-benaoxazin-2-one ; et
5) isolation de la 1,4-dihydro-2H-3,1-benzoxazin-2-one de la phase organique.

9. Procédé de préparation d'une 1,4-dihydro-2H-3,1-benzoxazin-2-one selon la revendication 8, dans lequel la base est définie comme un solide ou une solution de KOH, NaOH, LiOH, K₂CO₃, Na₂CO₃, Li₂CO₃, KHCO₃, NaHCO₃, LiHCO₃ ou un mélange desdites bases.

10. Procédé de préparation d'une 1,4-dihydro-2H-3,1-benzoxazin-2-one selon la revendication 9, dans lequel le solvant organique est choisi dans le groupe comprenant : oxyde de tert-butyle et de méthyle, toluène ou un mélange desdits solvants.

11. Procédé de préparation d'une 1,4-dihydro-2H-3,1-benzoxazin-2-one selon la revendication 10, dans lequel le groupe aryle du chloroformate d'aryle est défini comme le chloroformate de phényle, dans lequel le phényle peut être mono-, di- ou trisubstitué par des substituants choisis dans le groupe consistant en : halogènes (F, Cl, Br, I), CF₃ et NO₂.

12. Procédé de préparation d'une 1,4-dihydro-2H-3,1-benzoxazin-2-one selon la revendication 11, dans lequel la base est définie comme un solide ou une solution de KOH, NaOH, K₂CO₃, Na₂CO₃, KHCO₃, NaHCO₃ ou un mélange desdites bases.

13. Procédé de préparation d'une 1,4-dihydro-2H-3,1-benzoxazin-2-one selon la revendication 12, dans lequel le groupe aryle du chloroformate d'aryle est défini comme le 4-nitrophénylchloroformate.

14. Procédé de préparation d'une 1,4-dihydro-2H-3,1-benzoxazin-2-one de formule 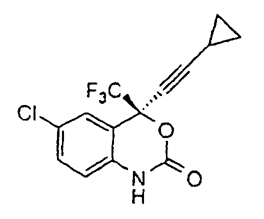 comprenant les étapes :
1) addition d'un chloroformate d'alkyle à un mélange en agitation d'un aminoalcool de formule 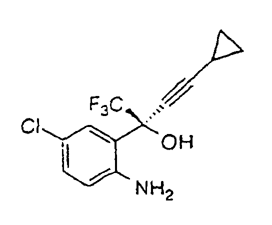 dans un premier solvant organique avec une première base, à une température allant d'environ 0 à environ 25 °C, sous atmosphère inerte, afin de produire un carbamate intermédiaire de formule 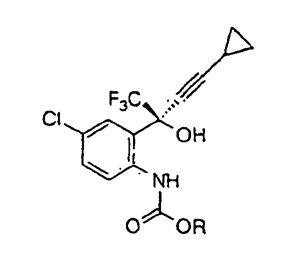 dans lequel R représente la chaîne latérale alkyle du chloroformate définie comme un alkyle en C₁ à C₁₀, pouvant être mono-, di- ou trisubstitué par des substituants choisis dans le groupe consistant en : CF₃, cycloalkyle en C₃ à C₇, alkoxycarbonyle en C₁ à C₆ et NO₂ ;
2) agitation du mélange réactionnel à une température allant d'environ 20 à environ 25°C pendant environ 1 à environ 30 heures afin d'achever la formation du carbamate intermédiaire ;
3) isolation de la phase organique contenant le carbamate d'alkyle;
4) distillation d'environ 90 à environ 95 % du premier solvant organique sous vide et addition d'un contre-solvant pour isoler le carbamate d'alkyle solide ;
5) addition d'un second solvant organique au carbamate d'alkyle solide pour former une solution de carbamate d'alkyle ;
6) réaction de la solution de carbamate d'alkyle avec une seconde base à une température allant d'environ 20 à environ 25 °C pendant environ 2 à environ 30 heures afin d'obtenir la 1,4-dihydro-2H-3,1-benzoxazin-2-one ;
7) refroidissement du milieu réactionnel à l'aide d'un acide afin d'obtenir une solution biphasique contenant la 1,4-dihydro-2H-3,1-benzoxazin-2-one en phase organique ;
8) isolation de la 1,4-dihydro-2H-3,1-benzoxazin-2-one de la phase organique.

15. Procédé de préparation d'une 1,4-dihydro-2H-3,1-benzoxazin-2-one selon la revendication 14, dans lequel la première base est définie comme un solide ou une solution de KOH, NaOH, LiOH, K₂CO₃, Na₂CO₃, Li₂CO₃, KHCO₃, NaHCO₃, LiHCO₃ ou un mélange desdites bases.

16. Procédé de préparation d'une 1,4-dihydro-2H-3,1-benzoxazin-2-one selon la revendication 15, dans lequel le premier solvant organique est choisi dans le groupe consistant en : oxyde de tert-butyle et de méthyle, toluène, tétrahydrofuranne, acétonitrile ou un mélange desdits solvants.

17. Procédé de préparation d'une 1,4-dihydro-2H-3,1-benzoxazin-2-one selon la revendication 16, dans lequel le contre-solvant est l'heptane.

18. Procédé de préparation d'une 1,4-dihydro-2H-3,1-benzoxazin-2-one selon la revendication 17, dans lequel le second solvant organique est choisi dans le groupe consistant en : oxyde de tert-butyle et de méthyle, toluène, tétrahydrofuranne, alcanol en C₁ à C₆ ou un mélange desdits solvants.

19. Procédé de préparation d'une 1,4-dihydro-2H-3,1-benzoxazin-2-one selon la revendication 18, dans lequel la seconde base est définie comme un solide ou une solution d'alkoxyde de potassium en C1 à C6, alkoxyde de sodium en C1 à C6, alkoxyde de lithium en C1 à C6, alkylpotassium en C1 à C6, alkylsodium en C1 à C6, alkyllithium en C1 à C6, KHMDS, NaHMDS, LiHMDS, LDA ou un mélange desdites bases.

20. Procédé de préparation d'une 1,4-dihydro-2H-3,1-benzoxazin-2-one selon la revendication 19, dans lequel l'acide est choisi dans le groupe consistant en : HCl, HNO₃, H₂SO₄ et CH₃CO₂H.

21. Procédé de préparation d'une 1,4-dihydro-2H-3,1-benzoxazin-2-one selon la revendication 20, comprenant en outre l'étape : cristallisation du carbamate d'alkyle synthétisé à l'étape 4 à partir de toluène et d'heptane ou d'oxyde de tert-butyle et de méthyle et d'heptane afin d'obtenir le carbamate d'alkyle désiré cristallisé.

22. Procédé de préparation d'une 1,4-dihydro-2H-3,1-benzoxazin-2-one selon la revendication 21, dans lequel le chloroformate d'alkyle est défini comme le chloroformate de méthyle ou d'éthyle.

23. Procédé de préparation d'une 1,4-dihydro-2H-3,1-benzoxazin-2-one selon la revendication 22, dans lequel le solvant organique (ou le mélange de solvants) est choisi dans le groupe consistant en : oxyde de tert-butyle et de méthyle, oxyde de tert-butyle et de méthyle et tétrahydrofuranne ou oxyde de tert-butyle et de méthyle et éthanol.

24. Procédé de préparation d'une 1,4-dihydro-2H-3,1-benzoxazin-2-one selon la revendication 23, dans lequel la base est définie comme un solide ou une solution de KHCO₃ et KOH, KHCO₃ et K₂CO₃ ou KHCO₃ et LiO^{t}Bu.

25. Un carbamate d'alkyle de formule : 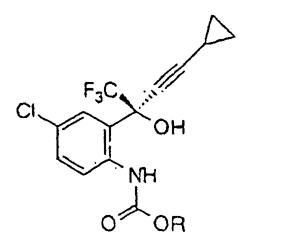 dans lequel R représente un alkyle en C₁ à C₁₀ qui peut être mono-, di- ou trisubstitué par des substituants choisis dans le groupe consistant en : CF₃, cycloalkyle en C₃ à C₇, alkoxycarbonyle en C₁ à C₆ et NO_{2.}
